# EUROPÄISCHE PATENTANMELDUNG

(11) **EP 4 382 068 A1**
(43) Veröffentlichungstag der Anmeldung: **12.06.2024**
(21) Anmeldenummer: 24164043.2
(22) Anmeldetag: 09.10.2018
(51) Int. Cl.: A61B 34/20, A61B 90/00, A61C 5/44, A61C 5/30, G02B 27/01

(54) **DENTALWERKZEUGSYSTEM**

(62) Teilanmeldung aus: 18199460.9
(71) Anmelder: Ivoclar Vivadent AG, 9494 Schaan (LI)
(72) Erfinder: Follonier, Stéphane, 7324 Viters (CH); Wachter, Wolfgang, 9494 Schaan (LI)
(74) Vertreter: Baldus, Oliver

(57) **Zusammenfassung**

Die vorliegende Erfindung betrifft ein Dentalwerkzeugsystem, mit einem Erfassungssystem (101) zum Erfassen einer räumlichen Position (103-Z) eines Zahnes (115) oder einer Zahnsituation und einer räumlichen Werkzeugposition (103-W) eines Werkzeuges (107); und einer Einrichtung (105) zum Steuern oder Regeln des Werkzeuges (107) auf Basis der erfassten Werkzeugposition (103-W) in Bezug zur Position (103-Z) des Zahnes (115) oder der Zahnsituation.

## Beschreibung

Die vorliegende Erfindung betrifft ein Dentalwerkzeugsystem und ein Verfahren zum Steuern eines Dentalwerkzeugs.

Dentalwerkzeuge zur zahnmedizinischen Behandlung, wie beispielsweise Bohrer oder Polymerisationslampen, können falsch bedient werden. Dies kann zu irreparablen Schäden während einer Zahnbehandlung bei einem Patienten führen.

Es ist daher die technische Aufgabe der vorliegenden Erfindung, eine Handhabung von steuerbaren Dentalwerkzeugen technisch zu vereinfachen.

Diese Aufgabe wird durch Gegenstände nach den unabhängigen Ansprüchen gelöst. Vorteilhafte Ausführungsformen sind Gegenstand der abhängigen Ansprüche, der Beschreibung und der Figuren.

Gemäß einem ersten Aspekt wird die vorliegende Aufgabe durch ein Dentalwerkzeugsystem gelöst, mit einem Erfassungssystem zum Erfassen einer räumlichen Position eines Zahnes oder einer Zahnsituation und einer räumlichen Werkzeugposition eines Werkzeuges; und einer Einrichtung zum Steuern oder Regeln des Werkzeuges auf Basis der erfassten Werkzeugposition in Bezug zur Position des Zahnes oder der erfassten Zahnsituation. Das Erfassen der Position des Zahnes oder der Zahnsituation kann mittels eines optischen Verfahrens oder eines Röntgenverfahrens durchgeführt werden. Im Allgemeinen kann das elektronische Erfassungssystem jedes Erfassungssystem sein, mit dem sich eine Position des Zahnes oder der Zahnsituation erhalten lässt.

Das Steuern oder Regeln kann erfolgen, indem ein räumlicher Abstand zwischen der Werkzeugposition und der Position des Zahnes oder der Zahnsituation berechnet wird. Das Steuern oder Regeln auf Basis der erfassten Werkzeugposition in Bezug zur Position des Zahnes oder der Zahnsituation kann beispielsweise erfolgen, falls die Werkezugposition außerhalb eines vorgegeben räumlichen Bereiches zur Position des Zahnes oder der Zahnsituation liegt oder falls der Abstand der Werkzeugposition zur Position des Zahnes oder der Zahnsituation einen vorgegebenen Abstand über- oder unterschreitet. Der Zahn zur Erfassung der Position des Zahnes oder der Zahnsituation kann über eine Benutzerschnittstelle ausgewählt werden. Das Erfassen der Position des Zahnes oder der Zahnsituation und der Werkzeugposition kann kontinuierlich in Echtzeit erfolgen. Durch das Dentalwerkzeugsystem wird beispielsweise der technische Vorteil erreicht, dass eine korrekte Verwendung des Dentalwerkzeugs in Bezug auf den zu behandelnden Zahn oder die zu behandelnde Stelle sichergestellt und eine positionsbasierte Steuerung des Werkzeuges durchgeführt werden kann.

Die Zahnsituation ist beispielsweise eine Situation des Zahnes oder der Zahnwurzel, die durch die Form, das Innere oder einen Kariesbefall gegeben ist. Im Allgemeinen kann die Zahnsituation durch jeden räumlichen Bereich des Zahnes gegeben sein, der der Bearbeitung durch das Werkzeug bedarf.

Beispielsweise kann ein Bohrer als Werkzeug auf Basis der Position der Zahnsituation eingeschaltet werden, solange dieser einen kariösen Bereich des Zahnes bearbeitet. Die Informationen über die Zahnsituation lassen sich beispielsweise mit einem Röntgenverfahren gewinnen. Die Tiefeninformation oder Röntgenbilder können die Position des Kariesbefalls im Zahn angeben.

In einer technisch vorteilhaften Ausführungsform des Dentalwerkzeugsystems ist eine optische Aufnahmeeinrichtung des elektronischen Erfassungssystems in eine Datenbrille integriert. Die Datenbrille ist ein als Brille getragenes elektronisches Gerät, mit dem einem Benutzer zusätzlich zur natürlichen visuellen Wahrnehmung weitere Informationen optisch angezeigt werden können. Die Datenbrille kann durch eine Anzeigeeinrichtung zusätzliche Informationen auf einem Bild überlagern, das von dem Auge des Trägers wahrgenommen wird. Die Anzeigeeinrichtung umfasst beispielsweise einen augennahen Bildschirm oder einen Projektor zur direkten Projektion auf der Netzhaut. Durch eine Integration des Erfassungssystems in die Datenbrille wird beispielsweise der technische Vorteil erreicht, dass sich der Aufbau des dentalen Führungssystems vereinfacht.

In einer weiteren technisch vorteilhaften Ausführungsform des Dentalwerkzeugsystems ist das Dentalwerkzeugsystem ausgebildet, einen Fehler optisch auf einer Anzeigeeinrichtung der Datenbrille anzuzeigen. Der Fehler ist beispielsweise ein Fehler auf Basis der erfassten Werkzeugposition in Bezug zur Position des Zahnes oder der Zahnsituation. Der Fehler entsteht beispielsweise, wenn das Werkzeug einen bestimmten Abstand zum Zahn unter- oder überschreitet. Dadurch wird beispielsweise der technische Vorteil erreicht, dass der Benutzer eine falsche Position des Werkzeuges unmittelbar erkennen kann.

In einer weiteren technisch vorteilhaften Ausführungsform des Dentalwerkzeugsystems umfasst das Erfassungssystem ein stereoskopisches Erfassungssystem mit einer ersten und einer zweiten Kamera. Dadurch wird beispielsweise der technische Vorteil erreicht, dass die Positionen mit geringem technischen Aufwand erfasst werden können und sich das Erfassungssystem auf einfache Weise in eine Datenbrille integrieren lässt.

In einer weiteren technisch vorteilhaften Ausführungsform des Dentalwerkzeugsystems umfasst das Erfassungssystem eine Kamera zum Erfassen der Position des Zahnes oder der Zahnsituation und der Werkzeugposition basierend auf einer Laufzeitmessung von Licht. Dadurch wird beispielsweise der technische Vorteil erreicht, dass sich die Positionen schnell und mit einer hohen Genauigkeit ermitteln lassen.

In einer weiteren technisch vorteilhaften Ausführungsform des Dentalwerkzeugsystems umfasst das Erfassungssystem eine Projektionseinrichtung zum Projizieren eines Lichtmusters. Das Erfassungssystem kann das projizierte Lichtmuster erfassen. Basierend auf dem erfassten Lichtmuster kann die räumliche Position des Zahnes oder der Zahnsituation berechnet werden. Dadurch wird beispielsweise der technische Vorteil erreicht, dass eine genaue Positionserfassung zu erreicht wird.

In einer weiteren technisch vorteilhaften Ausführungsform des Dentalwerkzeugsystems ist das Erfassungssystem ausgebildet, die Position des Zahnes oder der Zahnsituation und die Werkzeugposition auf Basis einer zeitlichen Abfolge von Bildern zu ermitteln. Dadurch wird beispielsweise der technische Vorteil erreicht, dass sich die Positionen des Zahnes oder der Zahnsituation und die Werkzeugposition auf einfache und genaue Weise ermitteln lassen. Jedes weitere Bild der Abfolge erhöht die Genauigkeit der ermittelten Position des Zahnes oder der Zahnsituation und Werkzeugposition. Hierzu können spezielle Computeralgorithmen verwendet werden.

In einer weiteren technisch vorteilhaften Ausführungsform des Dentalwerkzeugsystems ist das elektronische Erfassungssystem ausgebildet, eine Zeitdauer zu bestimmen, in der die Werkezugposition innerhalb eines vorgegeben räumlichen Bereiches zur Position des Zahnes oder der Zahnsituation liegt. Dadurch wird beispielsweise der technische Vorteil erreicht, dass eine Zeitsteuerung des Werkzeugs durchgeführt werden kann, beispielsweise kann das Werkzeug nach Ablauf einer vorgegebenen Zeitdauer innerhalb eines vorgegebenen räumlichen Bereiches abschaltet werden.

In einer weiteren technisch vorteilhaften Ausführungsform des Dentalwerkzeugsystems ist das elektronische Erfassungssystem ausgebildet, die Art des eingesetzten Werkzeugs optisch oder elektronisch zu erkennen. Die optische Erkennung des Werkzeugs erfolgt beispielsweise durch einen Vergleich eines aufgenommenen Bildes des Werkzeugs mit einem zuvor gespeicherten Bild des Werkzeugs. Die elektronische Erkennung des Werkzeugs erfolgt beispielsweise drahtlos mittels elektromagnetischer Wellen. Zu diesem Zweck kann das Werkzeug ein Sender-Empfänger-System zum automatischen und berührungslosen Identifizieren des Werkzeugs mit Radiowellen umfassen. Beispielsweise ist in dem Werkzeug ein RFID-Chip angeordnet, der das Werkzeug identifiziert und von einem Empfänger ausgelesen wird. Dadurch wird beispielsweise der technische Vorteil erreicht, dass auf Basis des erkannten Werkzeuges automatisch eine geeignete Steuerung ausgewählt werden kann.

In einer weiteren technisch vorteilhaften Ausführungsform des Dentalwerkzeugsystems umfasst das Werkzeug eine Polymerisationslampe, einen Zahnbohrer, ein Endoskop, eine Kamera, einen Scanner, ein Spiegel, ein Lichthärtegerät, ein Behandlungsstuhl, eine Lichtquelle zur Diagnostik oder eine Lichtquelle zur Farbebestimmung. Dadurch wird beispielsweise der technische Vorteil erreicht, dass zur Steuerung besonders geeignete Werkzeuge verwendet werden.

In einer weiteren technisch vorteilhaften Ausführungsform des Dentalwerkzeugsystems umfasst das Erfassungssystem eine Autofocus- oder Zoomfunktion zum Erfassen der Position des Zahnes oder der Zahnsituation und/oder Werkzeugposition. Die Autofocus-Funktion erlaubt eine automatische Scharfstellung, indem eine Kameraeinstellung an die Entfernung zwischen Kamera und Zahn oder Werkzeug angepasst wird und der Zahn oder das Werkzeug scharf abgebildet wird. Die Zoom-Funktion erlaubt eine stufenlose Anpassung des Bildausschnitts an den Zahn oder das Werkzeug. Dadurch wird beispielsweise der technische Vorteil erreicht, dass sich die Bedienung und Handhabung des Dentalwerkzeugsystems vereinfacht.

In einer weiteren technisch vorteilhaften Ausführungsform des Dentalwerkzeugsystems ist das Erfassungssystem ausgebildet, die räumliche Position des Zahnes oder der Zahnsituation basierend auf einer Abfolge von Zahnbildern des Zahnes ermitteln, die aus unterschiedlichen Blickwinkeln gewonnen worden sind. Dadurch wird beispielsweise der technische Vorteil erreicht, dass eine einfache und schnelle Positionserfassung zu erreicht wird.

Gemäß einem zweiten Aspekt wird die Aufgabe durch ein Verfahren zum Steuern eines Dentalwerkzeugs gelöst, mit den Schritten eines Erfassens einer räumlichen Position eines Zahnes oder einer Zahnsituation und einer räumlichen Werkzeugposition eines Werkzeuges; und eines Steuerns oder Regelns des Werkzeuges auf Basis der erfassten Werkzeugposition in Bezug zur Position des Zahnes oder der Zahnsituation. Das Steuern oder Regeln auf Basis der erfassten Werkzeugposition in Bezug zur Position des Zahnes oder der Zahnsituation kann beispielsweise erfolgen, falls die Werkezugposition außerhalb eines vorgegeben räumlichen Bereiches zur Position des Zahnes oder der Zahnsituation liegt. Durch das Verfahren werden die gleichen technischen Vorteile wie durch das Dentalwerkzeugsystem nach dem ersten Aspekt erreicht.

In einer technisch vorteilhaften Ausführungsform des Verfahrens wird ein Fehler optisch auf einer Anzeigeeinrichtung einer Datenbrille angezeigt. Dadurch wird beispielsweise ebenfalls der technische Vorteil erreicht, dass sich der Aufbau des Dentalwerkzeugsystems vereinfacht.

In einer weiteren technisch vorteilhaften Ausführungsform des Verfahrens werden die Position des Zahnes oder der Zahnsituation und die Werkzeugposition durch ein stereoskopisches Erfassungssystem oder durch ein Erfassungssystem basierend auf einer Laufzeitmessung von Licht erfasst. Dadurch wird beispielsweise ebenfalls der technische Vorteil erreicht, dass sich die Positionen schnell und mit einer hohen Genauigkeit ermitteln lassen.

In einer weiteren technisch vorteilhaften Ausführungsform des Verfahrens werden die Position des Zahnes oder der Zahnsituation und die Werkzeugposition auf Basis einer Abfolge von Bildern ermittelt. Dadurch wird beispielsweise ebenfalls der technische Vorteil erreicht, dass sich die Position des Zahnes oder der Zahnsituation und die Werkzeugposition auf einfache und genaue Weise ermitteln lassen.

In einer weiteren technisch vorteilhaften Ausführungsform des Verfahrens wird eine Zeitdauer bestimmt, in der die

Werkzeugposition innerhalb des vorgegeben räumlichen Bereiches zur Position des Zahnes oder der Zahnsituation liegt. Dadurch wird beispielsweise ebenfalls der technische Vorteil erreicht, eine Zeitsteuerung des Werkzeugs durchgeführt werden kann.

In einer weiteren technisch vorteilhaften Ausführungsform des Verfahrens wird die Art des eingesetzten Werkzeugs optisch durch das Erfassungssystem erkannt. Dadurch wird beispielsweise ebenfalls der technische Vorteil erreicht, dass auf Basis des erkannten Werkzeuges automatisch eine geeignete Steuerung ausgewählt werden kann.

Ausführungsbeispiele der Erfindung sind in den Zeichnungen dargestellt und werden im Folgenden näher beschrieben.

Es zeigen:
- Fig. 1: eine schematische Ansicht eines Dentalwerkzeugsystems;
- Fig. 2: eine Datenbrille zur Betrachtung für das Dentalwerkzeugsystem; und
- Fig. 3: ein Blockdiagram eines Verfahrens zum Steuern eines Dentalwerkzeugs.

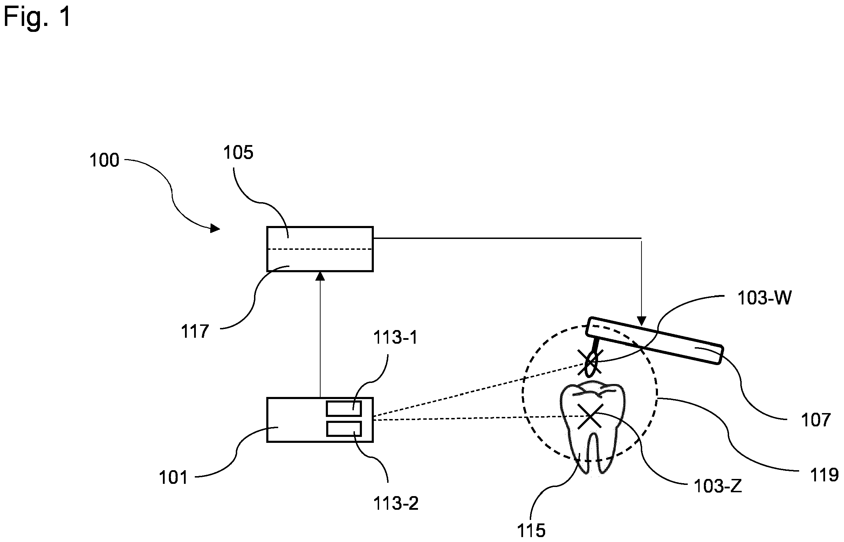

Fig. 1 zeigt eine schematische Ansicht eines Dentalwerkzeugsystems 100. Das Dentalwerkzeugsystem 100 dient zum Steuern oder Regeln eines Werkzeuges 107, das für eine zahnmedizinische Behandlung verwendet wird. Bei dem Werkzeug 107 kann es sich beispielsweise um eine Polymerisationslampe, einen Zahnbohrer, ein Endoskop, eine Kamera, einen Scanner, ein Spiegel, eine Lichtquelle zur Diagnostik oder eine Lichtquelle zur Farbbestimmung handeln. Im Allgemeinen kann das Werkzeug 107 jedoch jedes steuerbare Werkzeug sein, das im Rahmen einer zahnmedizinischen Behandlung eingesetzt wird. Die Steuerung des Werkzeugs 107 erfolgt auf Basis einer erfassten Werkzeugposition 103-W und einer Position 103-Z eines Zahnes 115 oder einer Zahnsituation. Die Position 103-Z des Zahnes 115 kann nicht nur die Position eines natürlichen Zahns sein, sondern ebenfalls die Position eines künstlichen Zahns, wie beispielsweise eine Brücke, eine Teilprothese, oder auch ein Implantat oder ein Abutment. Die Zahnsituation ist beispielsweise der Bereich eines Kariesbefalls oder der räumliche innere Bereich des Zahnes 115.

Die Werkzeugposition 103-W und die Position 103-Z des Zahnes 115 oder der Zahnsituation können durch ein optisches Erfassungssystem 101, ein Röntgenverfahren, ein Magnetresonanzverfahren oder ein Computertomographieverfahren erfasst werden.

Die Werkzeugposition 103-W oder die Position 103-Z können auch durch Funketiketten (RFID-Chips) erfasst werden, die im Werkzeug 107 oder dem Zahn 115 integriert sind. Die Werkzeugposition 103-W oder die Position 103-Z des Zahnes 115 oder der Zahnsituation können auch unter Verwendung eines Kalman-Filters bestimmt werden, der dazu beiträgt, der Berechnung der tatsächlichen Ist-Position des Werkzeugs 107 näher zu kommen. Das Kalman-Filter als mathematisches Verfahren überbrückt fehlende Messwerte oder kombiniert unterschiedliche Daten, wie optische Daten und Daten einer integrierten, inertialen Messeinheit (IMU). Im Allgemeinen kommen aber alle Verfahren in Frage, mit denen sich die Werkzeugposition 103-W oder die Position 103-Z des Zahnes 115 oder der Zahnsituation ermitteln lässt.

Das elektronische Erfassungssystem 101 erzeugt beispielsweise einen Datensatz, der die Position des Zahnes 115 als auch des Werkzeuges 107 umfasst oder beschreibt. Die Werkzeugposition 103-W und die Position 103-Z des Zahnes 115 oder der Zahnsituation können durch dreidimensionale Koordinaten in einem beliebigen Referenzkoordinatensystem angegeben sein. Die Position 103-Z des Zahnes 115 ist beispielsweise die Mitte des Zahnes 115 und die Werkzeugposition 103-W die Mitte eines Bohrkopfes. Im Allgemeinen kann die jeweilige Position jedoch auch durch andere Referenzpunkte gegeben sein, da sich zwischen den so definierten Koordinatensystemen auf einfache Weise umrechnen lässt.

Das elektronische Erfassungssystem 101 umfasst beispielsweise ein stereoskopisches Kamerasystem mit zwei Kameras 113-1, 113-2. Durch die stereoskopische Aufnahme mit den beiden Kameras 113-1, 113-2 lassen sich die Werkzeugposition 103-W und die Position 103-Z des Zahnes 115 oder der Zahnsituation auf einfache Weise ermitteln und rekonstruieren. Aus den jeweiligen Bildern der Kameras 113-1, 113-2 unter unterschiedlichen Parallaxenwinkeln oder durch einen Bildvergleich, lässt sich die Werkzeugposition 103-W und die Position 103-Z des Zahnes 115 oder der Zahnsituation mittels eines Computeralgorithmus berechnen.

Das Erfassungssystem 101 kann auch eine Kamera umfassen, die mit einem Laufzeitverfahren von Licht einzelne Distanzen ermittelt (TOF-Kamera - Time of Flight Kamera). Zu diesem Zweck wird die Mundhöhle mit dem Zahn 115 und dem Werkzeug 107 mittels eines Lichtpulses ausgeleuchtet und für jeden Bildpunkt die Zeit gemessen, die das Licht bis zum Objekt und wieder zurück braucht. Diese Zeit ist direkt proportional zur Distanz. Die Kamera liefert ein räumliches Modell und für jeden Bildpunkt die Entfernung des darauf abgebildeten Zahnes 115 und des Werkzeugs, so dass sich die jeweiligen Positionen des Zahnes 115 und des Werkzeuges 107 berechnen lassen.

Das Erfassungssystem 101 kann die Werkzeugposition 103-W und die Position 103-Z des Zahnes 115 oder der Zahnsituation auch basierend auf einer Abfolge von Zahnbildern des Zahnes 115 ermitteln, die aus unterschiedlichen Blickwinkeln gewonnen worden sind. Zu diesem Zweck kann ein Berechnungsverfahren verwendet werden, dass aus den einzelnen Bildern die Werkzeugposition 103-W als auch die Position 103-Z des Zahnes 115 oder der Zahnsituation rekonstruiert. Hierzu kann zusätzlich zur Werkzeugposition 103-W und Position 103-Z des Zahnes 115 oder der Zahnsituation eine Information über die räumliche Position des Erfassungssystems 101 verwendet werden.

Die ermittelte Werkzeugposition 103-W und die Position 103-Z des Zahnes 115 oder der Zahnsituation wird einer elektronischen Berechnungseinrichtung 117 als Datensatz zugeführt. Die Berechnungseinrichtung 117 kann beispielsweise ermitteln, ob die Werkzugposition 103-W außerhalb oder innerhalb eines vorgegeben räumlichen Bereiches 119 zur Position 103-Z des Zahnes 115 oder der Zahnsituation liegt. Der räumliche Bereich 119 kann automatisch auf Basis des verwendeten Werkzeuges 107 eingestellt werden. Der räumliche Bereich 119 kann beispielsweise kugelförmig um die Position 103-Z des Zahnes 115 oder der Zahnsituation herum vorgegeben sein. Im Allgemeinen kann der räumliche Bereich 119 auch beliebige andere räumliche Formen aufweisen. Beispielsweise kann der räumliche Bereich 119 bei der Verwendung einer Polymerisationslampe als Werkzeug 107 andere Abmessungen aufweisen, als bei der Verwendung eines Bohrers als Werkzeug 107.

Eine elektronische Steuerungseinrichtung 105 steuert das Werkzeug 107 auf Basis der erfassten Werkzeugposition 103-W in Bezug zur Position 103-Z des Zahnes 115 oder der Zahnsituation. Hierzu kann ein auf das Werkzeug 107 abgestimmtes Steuerungsschema verwendet werden. Zusätzlich kann eine Mitteilung an einen Benutzer des Dentalwerkzeugsystems 100 ausgegeben werden, wenn sich das Werkzeug 107 außerhalb oder innerhalb des vorgebeben räumlichen Bereiches 119 befindet. Zudem kann dem Benutzer eine haptische, akustische oder optische Rückmeldung über das Werkzeug 107 gegeben werden.

Das Dentalwerkzeugsystem 100 kann ausgebildet sein, eine elektronische Datei zu erzeugen, die einen Ablauf einer Behandlung dokumentiert, wie beispielsweise eine Videodatei oder CAD-Datei. In dieser Datei können die erfassten Werkzeugpositionen 103-W und die Positionen 103-Z des Zahnes 115 oder der Zahnsituation abgelegt sein. Dadurch können Informationen über die Behandlung dauerhaft elektronisch gespeichert werden, so dass im Nachhinein die Bearbeitung des Zahnes 115 auf Basis der elektronischen Datei verfolgt und begutachtet werden kann.

Die elektronische Berechnungseinrichtung 117 und die Steuerungseinrichtung 105 sind beispielsweise jeweils durch ein Software-Modul gebildet, das auf einer Computereinrichtung mit einem Prozessor und einem elektronischen Speicher zum Speichern des Softwaremoduls und der Datensätze ausgeführt wird. Zusätzlich können die Berechnungseinrichtung 117 oder die Steuerungseinrichtung 105 ausgebildet sein, eine Zahnform auf Basis von erfassten optischen Bildern zu berechnen. Die Zahnform ist beispielsweise die tatsächliche räumliche Form eines natürlichen Zahnes, einer Brücke oder einer Krone.

Handelt es sich bei dem Werkzeug 107 beispielsweise um eine Polymerisationslampe zum Aushärten einer Füllmasse, so kann diese automatisch ein oder ausgeschaltet werden, sobald sich diese im vorgegebenen räumlichen Bereich 119 um den Zahn 115 herum befindet. Dadurch kann sichergestellt werden, dass die Polymerisationslampe den korrekten Zahn 115 belichtet. Zudem kann eine Sicherheit der Behandlung verbessert werden und eine erhöhte Temperatur der Pulpa durch Polymerisationslampe vermieden werden. Zu diesem Zweck kann anhand der Werkzeugposition 103-W bestimmt werden, wie lange sich das Werkzeug 107 innerhalb des räumlichen Bereichs 119 befindet, d.h. eine Zeitdauer.

Handelt es sich bei dem Werkzeug 107 demgegenüber um einen Zahnbohrer, so kann dieser erst aktivierbar geschaltet sein, sobald sich dieser im vorgegebenen räumlichen Bereich 119 um den Zahn 115 herum befindet. Verlässt der Zahnbohrer den vorgegebenen räumlichen Bereich 119 um den Zahn 115 herum, so kann dieser automatisch abgeschaltet werden oder eine Drehgeschwindigkeit reduziert werden. Dadurch lassen sich Verletzungen durch den Zahnbohrer im Bereich der Mundhöhle verhindern.

Bei einem Lichthärtegerät als Werkzeug 107 kann erfasst werden, ob dieses an einer geeigneten Position gehalten wird, um das auszuhärtende Material auszuhärten. Das Steuern oder Regeln des Lichthärtegeräts auf Basis der erfassten Werkzeugposition in Bezug zur Position 103-Z des Zahnes 115 kann beispielsweise erfolgen, indem das Lichthärtegerät automatisch eingeschaltet wird, sobald dieses einen vorgegebenen Abstand zur Position 103-Z unterschreitet oder automatisch ausgeschaltet wird, sobald dieses einen vorgegebenen Abstand zur Position 103-Z unterschreitet. In gleicher Weise kann eine Zeitdauer oder Lichtintensität der Beleuchtung gesteuert werden. Bei dem Lichthärtegerät kann jedoch auch die Lichtintensität, die Wellenlänge oder ein Polarisationsgrad des ausgesandten Lichtes in Abhängigkeit der erfassten Werkzeugposition 103-W in Bezug zur Position 103-Z gesteuert werden. Verglichen mit dem Werkzeugzustand und der Zahn- oder Zahnfleischsituation können die Eigenschaften durch eine Messung der Wellenlänge und deren Auswirkung auf deren Umfeld ermittelt werden.

Bei einem Behandlungsstuhl als zahnmedizinisches Werkzeug 107 kann dieser in Abhängigkeit der Werkzeugposition in Bezug zur Position 103-Z verfahren oder verstellt werden, falls dieser zu weit entfernt ist oder Lichtverhältnisse nicht angemessen sind. Im Allgemeinen können aber auch andere Werkzeuge 107 gemäß deren Eigenschaften auf Basis der erfassten Werkzeugposition 103-W in Bezug zur Position 103-Z gesteuert werden.

Das Erfassungssystem 101 oder die Berechnungseinrichtung 117 können ausgebildet sein, die Art des eingesetzten Werkzeugs optisch zu erkennen. Die Art gibt beispielsweise an, um welchen speziellen Typ und um welches Model es sich bei dem eingesetzten Werkzeug 107 handelt. Beispielsweise kann die Art des Werkzeugs 107 anhand eines Bildvergleichs mit vorab gespeicherten digitalen Bildern erfolgen, beispielsweise mittels neuronaler Netze. Ansonsten könnten die Werkzeuge 107 auch mit einem miniaturisierten Bar- oder QR-Codes versehen sein, durch den die Art des Werkzeugs 107 erkannt werden kann. In Abhängigkeit der erkannten Art des Werkzeugs 107 kann wiederum der räumliche Bereich 119 für das Werkzeug 107 automatisch ausgewählt werden.

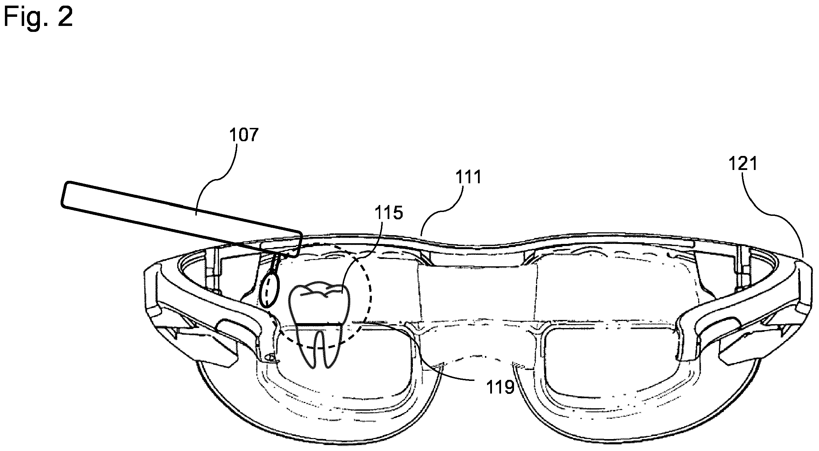

Fig. 2 zeigt eine Datenbrille 111 zur Betrachtung für das Dentalwerkzeugsystem 100. Eine oder mehrere optische Aufnahmeeinrichtungen des elektronischen Erfassungssystems 101 können in diese Datenbrille 111 intergiert sein. Dadurch erhöht sich die Handhabbarkeit des Dentalwerkzeugsteuerungssystems 100. Im Allgemeinen kann das Erfassungssystem 101 auch als separates Gerät vorgesehen sein, mit dem die Werkzeugposition 103-W und die Position 103-Z des Zahnes 115 oder der Zahnsituation ermittelt wird.

Die Datenbrille 111 (auch Augmented-Reality-Brille oder Smart-Glasses) ist ein tragbares Gerät, das in der Lage ist, virtuell Informationen vor die Augen des Brillenträgers zu projizieren, während dieser die Umwelt weiterhin visuell wahrnehmen kann. Dadurch können Informationen in dem Sichtfeld des Trägers angezeigt und hinzugefügt werden. Zu diesem Zweck umfasst die Datenbrille 111 eine Anzeigeeinrichtung 121, die aus einem augennahen Bildschirm oder einen Projektor zur direkten Projektion auf der Netzhaut gebildet sein kann.

Die Datenbrille 111 kann zusätzlich noch Sensoren zur Bewegungserfassung des Kopfes oder zur räumlichen Positionserkennung der Datenbrille 111 umfassen, wie beispielsweise einen Gyrosensor. Die räumliche Position der Datenbrille 111 kann aber auch auf Basis vorgegebener optischer Referenzpunkte erfolgen, die in der Umgebung der Datenbrille 111 angeordnet sind, wie beispielsweise mittels einer Trilateration oder einer Triangulation.

Dadurch kann die räumliche Position der Datenbrille 111 bei der Ermittlung und Berechnung der Werkzeugposition 103-W und der Position 103-Z des Zahnes 115 oder der Zahnsituation berücksichtigt werden, so dass die Genauigkeit der ermittelten Werkzeugposition 103-W und Position 103-Z des Zahnes 115 oder der Zahnsituation erhöht wird.

Die Datenbrille 111 kann zusätzlich den räumlichen Bereich 119 für das Werkzeug 107 anzeigen. Dieser räumlichen Bereich 119 kann optisch dem visuell wahrgenommenen Bild überlagert werden, beispielsweise durch eine farbliche Hervorhebung oder eine Strichlinie. Des Weiteren können Fehler oder Warnungen optisch auf der Datenbrille 111 bei der Verwendung des Werkzeugs 107 angezeigt werden.

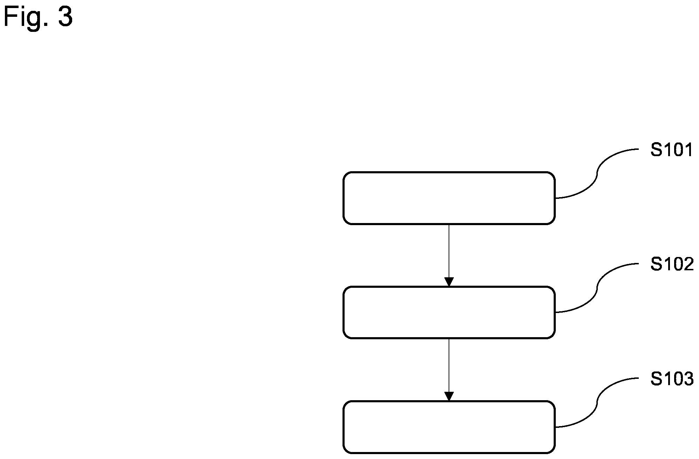

Fig. 3 zeigt ein Blockdiagram eines Verfahrens zum Steuern des Dentalwerkzeugs. Das Verfahren umfasst den Schritt S101 eines optischen Erfassens der räumlichen Position 103-Z des Zahnes 115 oder der Zahnsituation und einer räumlichen Werkzeugposition 103-W des Werkzeuges 107. Das Erfassen der räumlichen Position 103-Z des Zahnes 115 oder der Zahnsituation und der räumlichen Werkzeugposition 103-W kann kontinuierlich und in Echtzeit erfolgen. Anschließend wird in Schritt das Werkzeug 107 auf Basis der erfassten Werkzeugposition 103-W in Bezug zur Position 103-Z des Zahnes 115 oder der Zahnsituation gesteuert. Dadurch können Behandlungsfehler vermieden werden und die Handhabbarkeit von Werkzeugen 107 erhöht werden.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Dentalwerkzeugsystem
- 101: Erfassungssystem
- 103-Z: Position des Zahnes oder der Zahnsituation
- 103-W: Werkzeugposition
- 105: Einrichtung zum Steuern oder Regeln
- 107: Werkzeug
- 109: Aufnahmeeinrichtung
- 111: Datenbrille
- 113: Anzeigeeinrichtung
- 115: Zahn
- 117: Berechnungseinrichtung
- 119: räumlicher Bereich

## Patentansprüche

1. Dentalwerkzeugsystem, mit:
- einem Erfassungssystem (101) zum Erfassen einer räumlichen Position (103-Z) eines Zahnes (115) oder einer Zahnsituation und einer räumlichen Werkzeugposition (103-W) eines Werkzeuges (107); und
- einer Einrichtung (105) zum Steuern oder Regeln des Werkzeuges (107) auf Basis der erfassten Werkzeugposition in Bezug zur Position (103-Z) des Zahnes (115) oder der Zahnsituation.

2. Dentalwerkzeugsystem (100) nach Anspruch 1, wobei eine optische Aufnahmeeinrichtung (109) des elektronischen Erfassungssystems (101) in eine Datenbrille (111) intergiert ist.

3. Dentalwerkzeugsystem (100) nach Anspruch 2, wobei das Dentalwerkzeugsystem (100) ausgebildet ist, einen Fehler optisch auf einer Anzeigeeinrichtung (113) der Datenbrille (111) anzuzeigen.

4. Dentalwerkzeugsystem (100) nach einem der vorangehenden Ansprüche, wobei das Erfassungssystem (101) ein stereoskopisches Erfassungssystem mit einer ersten und einer zweiten Kamera (113-1, 113-2) umfasst.

5. Dentalwerkzeugsystem (100) nach einem der vorangehenden Ansprüche, wobei das Erfassungssystem (101) eine Kamera zum Erfassen der Position (103-Z) des Zahnes (115) oder der Zahnsituation und der Werkzeugposition (103-W) basierend auf einer Laufzeitmessung von Licht umfasst.

6. Dentalwerkzeugsystem (100) nach einem der vorangehenden Ansprüche, wobei das Erfassungssystem (101) ausgebildet ist, die Position (103-Z) des Zahnes (115) oder der Zahnsituation und die Werkzeugposition (103-W) auf Basis einer Abfolge von Bildern zu ermitteln.

7. Dentalwerkzeugsystem (100) nach einem der vorangehenden Ansprüche, wobei das elektronische Erfassungssystem (101) ausgebildet ist, eine Zeitdauer zu bestimmen, in der die Werkezugposition (103-W) innerhalb eines vorgegebenen räumlichen Bereiches (119) zur Position (103-Z) des Zahnes (115) oder der Zahnsituation liegt.

8. Dentalwerkzeugsystem (100) nach einem der vorangehenden Ansprüche, wobei das elektronische Erfassungssystem (101) ausgebildet ist, die Art des eingesetzten Werkzeugs (107) optisch zu erkennen.

9. Dentalwerkzeugsystem (100) nach einem der vorangehenden Ansprüche, wobei das Werkzeug (107) eine Polymerisationslampe, einen Zahnbohrer, ein Endoskop, eine Kamera, einen Scanner, ein Spiegel, ein Lichthärtegerät, ein Behandlungsstuhl, eine Lichtquelle zur Diagnostik oder eine Lichtquelle zur Farbebestimmung umfasst.

10. Dentalwerkzeugsystem nach einem der vorangehenden Ansprüche, wobei das Erfassungssystem (111) eine Autofocus- oder Zoomfunktion zum Erfassen der Position (103-Z) des Zahnes (115) oder der Zahnsituation und /oder Werkzeugposition umfasst.

11. Dentalwerkzeugsystem nach einem der vorangehenden Ansprüche, wobei das Erfassungssystem (111) ausgebildet ist, die räumliche Position (103-Z) des Zahnes (115) oder der Zahnsituation basierend auf einer Abfolge von Zahnbildern des Zahnes (115) ermitteln, die aus unterschiedlichen Blickwinkeln gewonnen worden sind.

12. Verfahren zum Steuern eines Dentalwerkzeugs, mit den Schritten:
- Erfassen (S101) einer räumlichen Position (103-Z) eines Zahnes (105) oder einer Zahnsituation und einer räumlichen Werkzeugposition (103-W) eines Werkzeuges (107); und
- Steuern oder Regeln des Werkzeuges (107) auf Basis der erfassten Werkzeugposition (103-W) in Bezug zur Position (103-Z) des Zahnes (115) oder der Zahnsituation.

13. Verfahren nach Anspruch 12, wobei ein Fehler optisch auf einer Anzeigeeinrichtung (113) einer Datenbrille (111) angezeigt wird.

14. Verfahren nach Anspruch 12 oder 13, wobei die Position (103-Z) des Zahnes (115) oder der Zahnsituation und die Werkzeugposition (103-W) durch ein stereoskopisches Erfassungssystem oder durch ein Erfassungssystem basierend auf einer Laufzeitmessung von Licht erfasst werden.

15. Verfahren nach einem der Ansprüche 12 bis 13, wobei die Position (103-Z) des Zahnes (115) oder der Zahnsituation und die Werkzeugposition (103-W) auf Basis einer Abfolge von Bildern ermittelt werden.

16. Verfahren nach einem der Ansprüche 12 bis 15, wobei eine Zeitdauer bestimmt wird, in der die Werkzeugposition (103-W) innerhalb eines vorgegeben räumlichen Bereiches (119) zur Position (103-Z) des Zahnes (115) oder der Zahnsituation liegt.

17. Verfahren nach einem der Ansprüche 12 bis 16, wobei die Art des eingesetzten Werkzeugs (107) optisch oder elektronisch durch das Erfassungssystem (101) erkannt wird.
